# EUROPEAN PATENT APPLICATION

(11) **EP 4 571 781 A1**
(43) Date of publication of application: **18.06.2025**
(21) Application number: 23215569.7
(22) Date of filing: 11.12.2023
(51) Int. Cl.: G16H 50/70, G16H 70/00, G16H 10/60, G16H 30/20, G16H 30/40, G16H 50/20

(54) **ENCODING A MODEL FOR PROCESSING USING A LARGE LANGUAGE MODEL**

(71) Applicant: Koninklijke Philips N.V., 5656 AG Eindhoven (NL)
(72) Inventor: WEBER, Frank Michael, Eindhoven (NL); EWALD, Arne, Eindhoven (NL); WEHLE, Simon, 5656AG Eindhoven (NL); HAHN, Artur, Eindhoven (NL); PETERS, Jochen, Eindoven (NL); KOLOKOLNIKOV, Georgii, Eindhoven (NL)
(74) Representative: Philips Intellectual Property & Standards

(57) **Abstract**

According to an aspect, there is provided a computer-implemented method (200) for encoding an anatomical model of an anatomical structure of a subject into a data structure suitable for processing using a large language model, LLM, algorithm, the method comprising obtaining (202) an anatomical model of the anatomical structure of the subject, the anatomical model comprising a plurality of model elements; converting (204) a first set of model elements of the plurality of model elements of the anatomical model into a first vector representation capable of being processed using an LLM algorithm; and composing (206) a data structure representative of the plurality of model elements of the anatomical model based on the first vector representation; wherein the data structure comprises an encoded version of the anatomical model of the anatomical structure configured to be processed using the LLM algorithm.

## Description

### FIELD OF THE INVENTION

The invention relates generally to large language models (LLMs) and, more particularly, to encoding an anatomical model so that it can be processed by an LLM.

### BACKGROUND OF THE INVENTION

In the medical domain, it is common to use 3-dimensional anatomical models to represent part or all of an organ or a body part of a subject's body. Such anatomical models may be used, for example, for performing a medical assessment or medical analysis of the organ or body part. In some scenarios, an anatomical model may be viewed by a medical professional while they perform a medical procedure or intervention, such as a surgical procedure. In some examples, an underlying medical image may be displayed and/or manipulated using information derived from the anatomical model.

Interactions with anatomical models, such as manipulating a view of the anatomical model by translation or rotation, for example, may typically be achieved using a user input device, such as a mouse, a keyboard and/or a touch screen device. However, such input devices generally require an operator's or user's hand to operate the input device to manipulate the view of the anatomical model, meaning that their hand cannot be used in any ongoing medical procedure.

A large language model (LLM) is a type of artificial neural network that has recently been developed in the field of artificial intelligence, and which is able to repeatedly predict the next word or token based on some input text. Speech-to-text translation allows an input of an LLM to be provided in the form of speech, meaning a user can interact with such an LLM in a hands-free manner.

While early LLM implementations were primarily text-based, more recent implementations include relations with images, meaning that image content can be identified, and in some cases generated, based on an input containing words and phrases. Similarly, an LLM is capable of generating an output of words and phrases given an input of an image. However, complex anatomical structures cannot be captured by a single two-dimensional image and, therefore, there is a need to be able to use LLM is to interact with representations of complex anatomical structures.

### SUMMARY OF THE INVENTION

It would be desirable to be able to use an LLM algorithm to interact with (e.g., manipulate a view of) a three-dimensional model of an anatomical structure in order to overcome or at least mitigate some of the issues identified above.

The inventors of the present disclosure have recognised that, by including an anatomical model of an anatomical structure in a particular manner, manipulation by an LLM algorithm is possible. Specifically, according to various embodiments disclosed herein, an anatomical model of an anatomical structure may be encoded into a data structure having at least one vector representation that can be processed using an LLM algorithm. In this way, the encoded version of the anatomical model can also be processed using the LLM algorithm, meaning that a user can use the LLM algorithm in conjunction with the encoded version of the anatomical model, for example to view, manipulate, analyse or modify the anatomical model.

According to a first specific aspect, there is provided a computer-implemented method for encoding an anatomical model of an anatomical structure of a subject into a data structure suitable for processing using a large language model, LLM, algorithm, the method comprising:
obtaining an anatomical model of the anatomical structure of the subject, the anatomical model comprising a plurality of model elements; converting a first set of model elements of the plurality of model elements of the anatomical model into a first vector representation capable of being processed using an LLM algorithm; composing a data structure representative of the plurality of model elements of the anatomical model based on the first vector representation; wherein the data structure comprises an encoded version of the anatomical model of the anatomical structure configured to be processed using the LLM algorithm.

In some embodiments, the method may further comprise converting a second set of model elements of the plurality of model elements of the anatomical model into a second vector representation capable of being processed using an LLM algorithm. Composing the data structure representative of the plurality of model elements of the anatomical model may comprise concatenating the first vector representation and the second vector representation.

The method may further comprise obtaining, from at least one of: i) a user input; and ii) the anatomical model, at least one data string to be included in the data structure, the at least one data string being associated with the anatomical model.

In some embodiments, the method may further comprise appending the at least one data string as a header of the first vector representation.

The at least one data string may comprise an indication of the nature of the first vector representation in the data structure. In some embodiments, the at least one data string may comprise data indicative of a type of anatomical structure represented by the anatomical model.

In some embodiments, the method may further comprise converting the at least one data string into an additional vector representation capable of being processed using an LLM algorithm. The at least one data string may comprise one or more offset values to distinguish the first vector representation from the additional vector representation in the data structure.

According to a second specific aspect, there is provided an apparatus for encoding an anatomical model of an anatomical structure of a subject into a data structure suitable for processing using a large language model, LLM, algorithm, the apparatus comprising:
a processor configured to perform a method as disclosed herein.

According to a third specific aspect, there is provided a data structure comprising information describing an anatomical model, wherein the information is encoded into a first vector representation for processing using a large language model, LLM, algorithm. The first vector representation comprises a plurality of vectors, each vector representing a corresponding one of a plurality of model elements of the anatomical model.

According to a fourth specific aspect, there is provided a computer-implemented method of processing a data structure using a large language model, LLM, algorithm, the method comprising obtaining input data, the input data comprising (i) one or more elements of natural language and (ii) a data structure as disclosed herein; and processing the input data using an LLM algorithm to generate output data.

According to a fifth specific aspect, there is provided a computer-implemented method of processing a data structure using a large language model, LLM, algorithm, the method comprising: obtaining input data comprising one or more elements of natural language; processing the input data with an LLM algorithm to generate output data, wherein the LLM algorithm has been trained using training data comprising a plurality of data structures as disclosed herein, each data structure describing a respective anatomical model.

The output data may comprise at least one of (i) a data structure describing an anatomical model; ii) a modified data structure describing the anatomical model and (iii) one or more elements of natural language.

According to a sixth specific aspect, there is provided a computer-implemented method of processing a data structure using a large language model, LLM, algorithm, the method comprising obtaining input data, the input data comprising a data structure as disclosed herein; and processing the input data with an LLM algorithm to generate output data comprising at least one of (i) a modified data structure describing the anatomical model; and (ii) one or more elements of natural language.

In some embodiments, the method may further comprise generating, for presentation to a user, a rendering based on the output data.

According to a seventh specific aspect, there is provided a computer-implemented method of training a large language model, LLM, algorithm, the method comprising obtaining input training data comprising a plurality of data structures as disclosed herein; creating training data from the input training data; and training the LLM algorithm using the training data to obtain a trained LLM algorithm.

In some embodiments, the training data further may comprise a plurality of training inputs and a respective plurality of corresponding training outputs, wherein each training input comprises (i) one or more first elements of natural language and (ii) a data structure describing an anatomical model, and wherein the corresponding training output comprises (i) one or more second elements of natural language and/or (ii) a modified data structure describing the anatomical model.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of an example of a system for encoding an anatomical model;
Fig. 2 is a flowchart of an example of a method of encoding an anatomical model;
Fig. 3 is a schematic illustration of an example of an anatomical model and a table representing parts of the anatomical model;
Fig. 4 is a flowchart of a further example of a method of encoding an anatomical model;
Fig. 5 is a schematic illustration of an example of an apparatus for encoding anatomical model;
Fig. 6 is a schematic illustration of an example of a data structure;
Fig. 7 is a flowchart of an example of a method of processing a data structure;
Fig. 8 is a flowchart of a further example of a method of processing a data structure;
Fig. 9 is a flowchart of a further example of a method of processing a data structure; and
Fig. 10 is a flowchart of a further example of a method of processing a data structure.

### DETAILED DESCRIPTION OF EMBODIMENTS

Various embodiments disclosed herein provide a mechanism by which an anatomical model of an anatomical structure may be encoded in such a way that it can be processed using a large language model (LLM) algorithm. Typically, LLMs process inputs in the form of words, sentences or other elements of natural language and may, for example, generate outputs that are also in the form of words, sentences or other elements of natural language. However, according to the present disclosure, and LLM may be used to process an anatomical model, either by receiving the anatomical model as an input or by generating the anatomical model, part of the anatomical model, or a modified version of the anatomical model as an output.

An LLM algorithm is an artificial neural network that has been trained using large amounts of training data to learn about syntax and semantics in language. An LLM algorithm is capable of recognising, translating, predicting and/or generating text or other content and is, therefore, capable of responding to a prompt (e.g., an input) with a response (e.g., output) which may, for example, be in the form of a word or a body of text. LLMs may include multiple neural network layers including, for example, one or more embedding layers, feedforward layers and/or recurrent layers.

Various types of LLM algorithm exist and may be used in the embodiments disclosed herein. A transformer model is an example of an architecture used in an LLM. A transformer model, which consists of an encoder and decoder, processes data by token rising an input, and using mathematical equations to discover relationships between tokens.

Embodiments disclosed herein relate to the encoding of an anatomical model of an anatomical structure so that the encoded version of the anatomical model can be processed using an LLM algorithm. As is discussed in greater detail below, part of the anatomical model may be converted into a vector representation that is capable of being processed using an LLM algorithm. For example, part of the anatomical model may be converted into a vector representation having the same or a similar format as other inputs (e.g., elements of natural language) that can be processed using the LLM algorithm. In other words, the anatomical model may be encoded so that it is in the same form as other data that can be processed using the LLM algorithm.

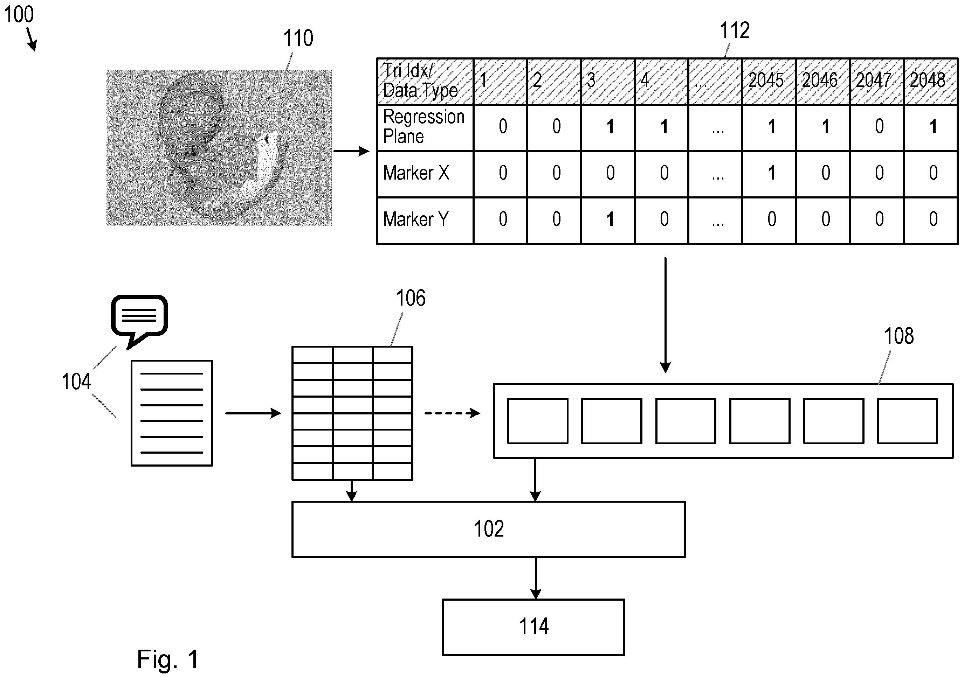

Referring now to the drawings, Fig. 1 is a schematic illustration of an example of a system 100 including a large language model algorithm 102. The LLM algorithm 102 may receive elements of natural language 104, including text (e.g., words, sentences and punctuation) and/or speech. It may be assumed that elements of speech (e.g., spoken word) may be translated into written text using a speech-to-text mechanism, such that the speech is translated into text before being provided as an input to the LLM algorithm 102. The elements of natural language 104 may be converted into a language vector representation 106 having a form that is capable of being processed using the LLM algorithm 102. Typically, the language vector representation 106 may be provided as an input to the LLM algorithm 102 for processing. However, according to some embodiments disclosed herein, the language vector representation 106 may be provided to a data structure 108 which can be used as an input for the LLM algorithm 102. As it is apparent from the discussion below, however, in some embodiments, the elements of natural language 104, and hence the language vector representation 106, may not be provided as an input to the LLM algorithm 102 and, therefore, may not be included in the data structure 108. Thus, in some embodiments, both the language vector representation 106 and a separate data structure 108 may be provided as inputs to the LLM algorithm 102 for processing, while in other embodiments, the language vector representation may be included in the data structure provided as an input.

The system 100 also includes an anatomical model 110 which may, for example, comprise a three-dimensional model of an organ (or part thereof), a body part (or part thereof), or the like, of a human or animal subject. The anatomical model 110 may be in any suitable form, such as a mesh model as shown in Fig. 1. A mesh model is a topological structure used to represent part of a subject's anatomy. Such a model may be expressed in terms of a vector of points and a set of connecting triangles that represent a surface, where each triangle represents a fixed location in the anatomical space. In general, the present disclosure relates to a mechanism by which the anatomical model 110 may be converted into a vector representation 112, which is included in the data structure 108. The data structure 108 is then provided as an input to the LLM algorithm 102. The data structure 108 (and, in some examples, also the vector representation 112) may be in the same form as the language vector representation 106. The vector representation 112 is included in the data structure 108, which is provided as an input to the LLM algorithm 102. Processing of the LLM algorithm 102 may generate an output 114 as discussed herein.

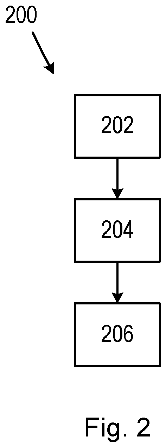

According to a first aspect, the present invention provides a method. Fig. 2 is a flowchart of an example of a method (e.g., a computer-implemented method) 200 for encoding an anatomical model (e.g., the anatomical model 110) of an anatomical structure of a subject into a data structure (e.g., the data structure 108) suitable for processing using a large language model (LLM) algorithm (e.g., the LLM algorithm 102). The method 200 comprises, at step 202, obtaining an anatomical model of the anatomical structure of the subject, the anatomical model comprising a plurality of model elements. As noted above, the anatomical model may be in any suitable form, such as a three-dimensional mesh model and may comprise a model of part or all of the anatomical structure of the subject. Each of the model elements of the plurality of model elements may comprise an elemental part of the anatomical model such as a shape defined by a set of vertices, or an individual triangle portion of the mesh anatomical model 110 shown in Fig. 1. In other examples, a model element may comprise a larger part of the anatomical model, such as a set of triangles or a more complex structure. It will be appreciated that in other examples, each model element may have some other shape, depending on the format of the anatomical model. In general, any model element may be used that can be encoded into a vector representation.

Obtaining the anatomical model in step 202 may for example involve retrieving the anatomical model from a storage device (e.g., a memory) or receiving the anatomical model as an input provided by a user or from elsewhere.

At step 204, the method 200 comprises converting a first set of model elements of the plurality of model elements of the anatomical model into a first vector representation capable of being processed using an LLM algorithm. The first set of model elements may comprise a subset of the plurality of model elements or all of the model elements in the plurality of model elements. The method 200 comprises, at step 206, composing a data structure representative of the plurality of model elements of the anatomical model based on the first vector representation. In other words, the first vector representation may be used as or incorporated into a data structure that represents the plurality of model elements. Examples of the data structure are discussed below, but it will be appreciated that the data structure may comprise any suitable data structure capable of being provided to and processed using an LLM algorithm. Thus, the data structure comprises an encoded version of the anatomical model of the anatomical structure configured to be processed using the LLM algorithm.

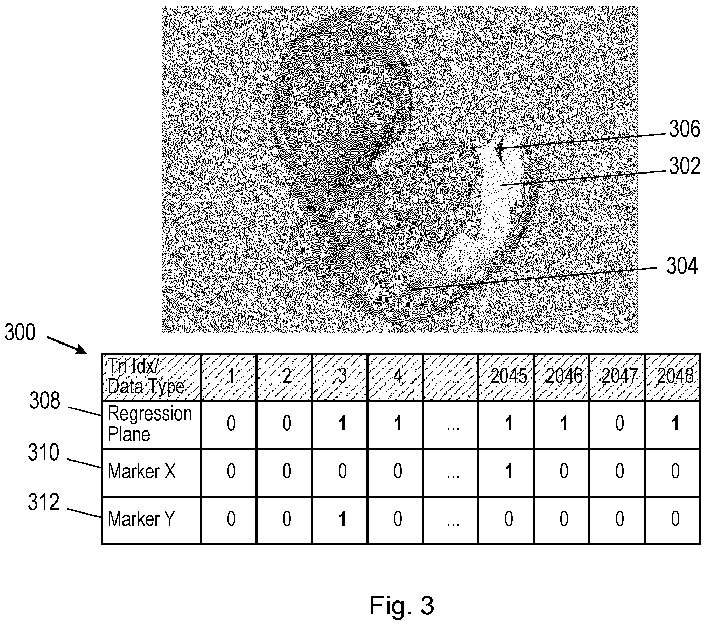

An example of the method 200 will now be discussed in greater detail with reference to Fig. 3. Fig. 3 is a schematic illustration of an example of the anatomical model 110 and a table 300 representing parts of the anatomical model. In this example, the anatomical model 110 is a three-dimensional mesh model of part of a human's heart. The anatomical model 110 is constructed using a plurality of triangles in this example, though it will be appreciated that other shapes or formations could be used to form the anatomical model. Thus, in this example, a triangle of the plurality of triangles may be considered to be a model element of the anatomical model 110. The anatomical model 110 includes a plane 302 (e.g., an anatomical plane) formed by a plurality of shaded triangles. The plane 302 may, for example, comprise a region of interest, and it may be intended that a viewer (e.g., a medical professional) is able to view, examine and analyse the plane in greater detail, for example by adjusting a position and/or orientation of the plane and/or the anatomical model 110 in a representation presented to them. For example, in Fig. 3, the plane 302 formed by the plurality of triangles is a plane through a particular region (e.g., a vein) of the right atrium of the heart (i.e., the anatomical structure). The plane 302 includes a first orientation marker 304 and a second orientation marker 306, defining an orientation of the anatomical model 110.

To enable this information about the anatomical model 110 to be processed by the LLM model, details about the anatomical model, the plane 302 and the first and second orientation marker 304, 306 are converted into at least one vector representation (step 204). The table 300 shows an example of how model elements (e.g., triangles in this example) of the anatomical model 110 may be represented as vectors. The table 300 includes 2048 bits of information for each of three rows defining parts of the anatomical model 110. Each bit of information represents one element of the plurality of elements of the anatomical model 110 so, in this case, each bit of the 2048 bits of information represents a triangle in the triangle mesh model. A first row 308 in the table 300 indicates whether or not each bit lies within the plane 302 which, in this example, comprises a regression plane. A second row 310 in the table 300 indicates whether or not each bit is the first orientation marker 304, and a third row 312 in the table indicates whether or not each bit is the second orientation marker 306. The table 300 is populated in a binary manner, with a '1' indicating a positive and a '0' indicating a negative. Thus, the data in each row 308, 310, 312, or the data in the entire table 300, may be converted into a vector representation or multiple vector representations, which can then be included in a data structure. Encoding the anatomical model 110 in this way makes it possible for an LLM algorithm to process the anatomical model, meaning that the benefits available through using an LLM algorithm can be applied in the medical field, particularly in the context of an anatomical model.

The example given above is for encoding the information about the anatomical model in a particular LLM encoding space, the size of which may depend on the LLM algorithm being used. In an example, the LLM encoding space may comprise 512 elements. If each element is a float value, then such an LLM encoding space may be able to store 512 x 32 bits.

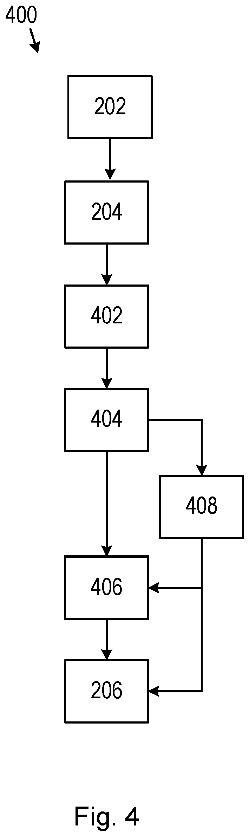

Fig. 4 is a flowchart of a further example of a method 400 of encoding an anatomical model. The method 400 includes steps of the method 200 discussed above. At step 402, the method 400 comprises converting a second set of model elements of the plurality of model elements of the anatomical model into a second vector representation capable of being processed using an LLM algorithm. Thus, in the example shown in Fig. 3, the first set of model elements may comprise a first set of triangles of the triangle mesh and the second set of model elements may comprise a second set of triangles of the triangle mesh. Each of the first and second set of model elements may be converted into its own vector representation. In examples where a second vector representation is created, composing the data structure representative of the plurality of model elements of the anatomical model (step 206) may comprise concatenating the first vector representation and the second vector representation. In other examples, additional sets of model elements may be converted into one or more additional vector representations, and composing the data structure may comprise concatenating the additional vector representations with the first and second vector representations.

In addition to the first vector representation defining the anatomical model, and any additional vector representations that may be available, the data structure may, in some embodiments, include additional information, for example in the form of one or more data strings. Thus, at step 404, the method 400 may comprise obtaining, from at least one of: i) a user input; and ii) the anatomical model, at least one data string to be included in the data structure, the at least one data string associated with the anatomical model. The at least one data string may, therefore, comprise information defining or describing some aspect of the anatomical model and/or of the plurality of model elements. In some embodiments, the at least one data string may be obtained through a user providing the data string via a user interface. In other embodiments, the at least one data string may form part of the anatomical model, and therefore may be obtained when the anatomical model is obtained at step 202 of the method 200.

In some embodiments, the at least one data string may comprise an indication of the nature of the first vector representation in the data structure. In other words, the at least one data string may comprise an indication of a meeting of the first vector representation and/or other vector representations included in the data structure. For example, the data string may identify a plane that a particular bit forms part of (e.g., a regression plane, a central plane, a plane in the x orientation, a plane and the y orientation, or the like).

In some embodiments, the at least one data string may comprise data indicative of a type of anatomical structure represented by the anatomical model. For example, the at least one data string may identify the body part, organ or anatomical structure represented by the anatomical model (e.g., heart, brain, liver or the like). Each data string of the at least one data string may be formed of any number of bits, depending on the nature (e.g., size) of the data structure in which each data string is to be included. In some examples, the at least one data string may comprise 32 bits.

One or more data strings of the at least one data string may be included in a vector representation (e.g., the first vector representation) as a header. Thus, the method 400 may comprise, at step 406, appending the at least one data string as a header of the first vector representation.

In some embodiments, the at least one data string may be included in the data structure in the format in which it is obtained or provided. In other embodiments, however, one or more data strings of the at least one data string may be converted into a format similar to the format of the model elements of the anatomical model (e.g., a vector representation). Thus, the method 400 may comprise, at step 408, converting the at least one data string into an additional vector representation capable of being processed using an LLM algorithm. For example, the at least one data string may be converted into an additional vector representation having a format similar to or the same as the first vector representation. In some embodiments, the at least one data string may comprise one or more offset values to distinguish the first vector representation from the additional vector representation in the data structure. In this way, the vector representations in the data structure are unique, and the risk of confusion between a vector representation defining the anatomical model with any other vector representation in the data structure is reduced.

Steps of the methods 200, 400 disclosed herein may be performed using one or more processors, for example a processor of a computing device or server.

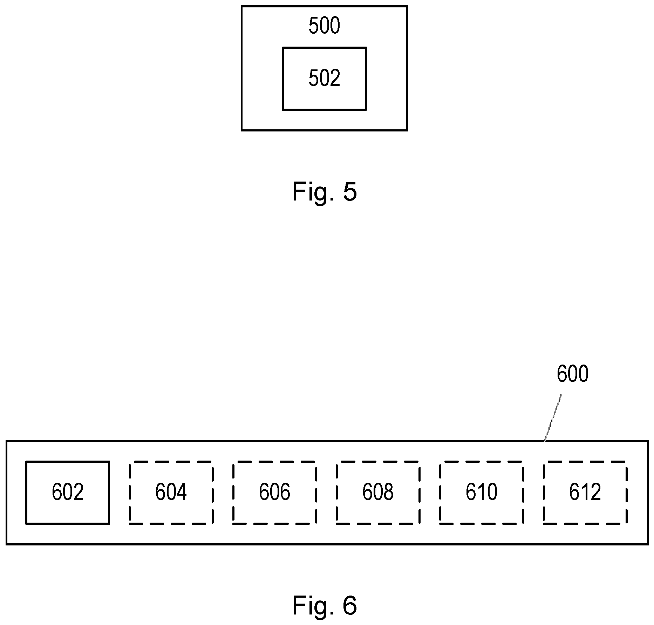

According to a further aspect, the present invention provides an apparatus. Fig. 5 is a schematic illustration of an example of an apparatus 500. The apparatus 500 is for encoding an anatomical model of an anatomical structure of a subject into a data structure suitable for processing using a large language model, LLM, algorithm. The apparatus 500 comprises a processor 502 configured to perform steps of at least one of the methods 200, 400 disclosed herein. The apparatus 500 may, for example, comprise a computing device such as a desktop computer, laptop computer, a tablet computer, a smart phone or a wearable device, or may comprise a component of a cloud-computing environment, such as a server.

According to a further aspect, the present invention provides a data structure. Fig. 6 is a schematic illustration of an example of a data structure 600. The data structure 600 comprises information describing an anatomical model, wherein the information is encoded into a first vector representation 602 for processing using a large language model, LLM, algorithm. The first vector representation 602 may comprise or be similar to the vector representation 112 discussed above or the LLM algorithm may comprise or be similar to the LLM algorithm 102 discussed above. The first vector representation comprises a plurality of vectors, each vector representing a corresponding one of a plurality of model elements of the anatomical model for example, each vector in the first vector representation may correspond to a triangle of a triangle mesh model, a set of triangles of a triangle mesh model, a plane of a triangle mesh model, or the like.

As discussed above, the data structure 600 may comprise components other than the first vector representation defining the anatomical model. For example, the data structure 600 may comprise a second vector representation 604 containing more information describing the anatomical model. In some embodiments, the data structure 600 may comprise a data string/vector representation 606 containing an indication of the nature of one or more of the vector representations (e.g., 602, 604) of the data structure. In some embodiments, the data structure 600 may comprise a data string/vector representation 608 containing data indicative of a type of anatomical structure represented by the anatomical model. In some embodiments, the data structure 600 may comprise one or more padding components 610 to fill space in the data structure (e.g., encoding space). In some embodiments, the data structure 600 may comprise a data string/vector representation 612 comprising one or more offset values to help distinguish components in the data structure (e.g., components 604, 606, 608, 610) from the first vector representation 602.

One or more of the data strings/vector representations and 606, 608 of the data structure 600 may be appended as a header of the first vector representation 602 and/or the second vector representation 604.

The data structure 600 may be configured (e.g., in terms of its size) according to the LLM algorithm that is to process the data structure. In some embodiments, for example, the data structure 600 may have a size (e.g., an encoding space) of 512 x 32 bits. In an example, the first vector representation 602 may have a size of 2048 bits (e.g., corresponding to the anatomical model having 2048 model elements, or triangles), the data strings/vector representations 606 and 608 may each have a size of 32 bits, and the rest of the data structure and may be filled with the one or more padding components 610 and/or the offset values. In other examples, a vector representing elements of natural language may be included in the data structure. In other examples, the data structure may have a size of N x 512 x 32 bits to contain a plurality of N individual elements that can be processed by the LLM algorithm.

The data structure may be considered to be a computer-readable medium storing information describing an anatomical model, wherein the information is encoded into a representation (e.g., the first vector representation) for processing using an LLM algorithm, wherein the representation comprises a plurality of vectors, each vector representing a corresponding one of a plurality of model elements of the anatomical model. The representation may be the same type as used to encode elements of natural language that can be processed using the LLM, to thereby enable combined processing of the encoded information with the elements of natural language using the LLM algorithm.

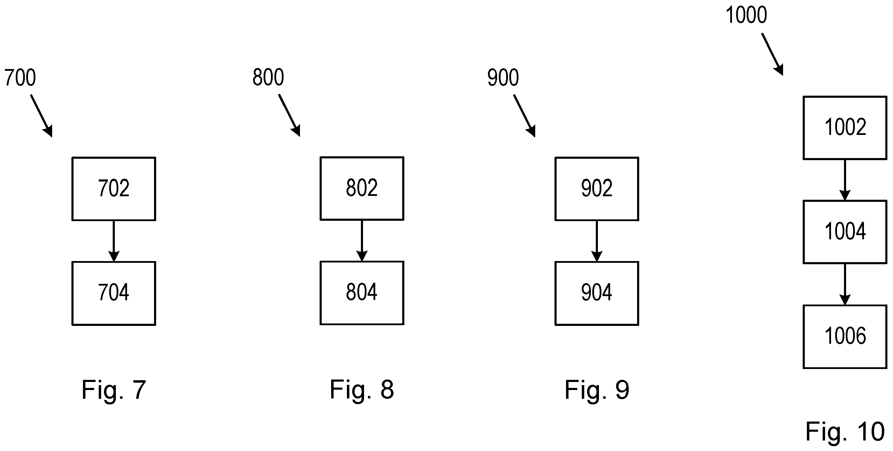

Further aspects of the invention provide methods of processing the data structure (e.g., the data structure 600) using an LLM algorithm 102. As is clear from the discussion below, the data structure 600 may be processed using the LLM algorithm 102 in various ways, with the data structure being provided as an input and/or being output by the LLM algorithm. Various methods of processing the data structure are discussed below with reference to Figs. 7, 8 and 9.

Fig. 7 is a flowchart of an example of a computer-implemented method 700 of processing a data structure using an LLM algorithm. The method 700 comprises, at step 702, obtaining input data, the input data comprising (i) one or more elements of natural language and (ii) a data structure as discussed herein. The one or more elements of natural language may comprise words, sentences, punctuation, or the like, provided in the form of text. At step 704, the method 700 comprises processing the input data using an LLM algorithm to generate output data. The output data may be in any suitable form, depending on the nature and purpose of the LLM algorithm and the processing. As an example, the data structure 600, including the first vector representation defining the anatomical model, maybe provided as an input to the LLM algorithm 102. The natural language provided as a further input to the LLM algorithm 102 may, for example, include a question such as "which anatomical structure is shown?", and the LLM algorithm 102 may generate an output describing the anatomical structure shown in the anatomical model. The output may be presented as an audible or visible output, for example on a display screen. In another example, the natural language provided as an input to the LLM algorithm 102 may include an instruction such as "shift plane more posterior", and the LLM algorithm 102 may generate an output that includes a modified version of the anatomical model in which the plane has been shifted posteriorly. In another example, the anatomical model may be aligned with an image dataset, such as a dataset containing one or more images of parts of the anatomical structure. In an example, an anatomical model may be updated (e.g., as an output of the LLM algorithm), and the dataset containing one or more images or a display of said dataset may be updated accordingly. In some examples, one or more cutting planes through the image or images, or one or more volume renderings, may be generated for display to a user, and/or an updated viewing location and direction for a three-dimensional rendering of the image may be provided.

Fig. 8 is a flowchart of a further example of a computer-implemented method 800 of processing a data structure using a large language model, LLM, algorithm. The method 800 comprises, at step 802, obtaining input data comprising one or more elements of natural language. As noted above, the one or more elements of natural language may comprise words, sentences, punctuation, or the like, provided in the form of text. At step 804, the method 800 comprises processing the input data with an LLM algorithm to generate output data, wherein the LLM algorithm has been trained using training data comprising a plurality of data structures as disclosed herein, each data structure describing a respective anatomical model.

In either of the methods 700, 800, the output data may comprise at least one of (i) a data structure describing an anatomical model; ii) a modified data structure describing the anatomical model and (iii) one or more elements of natural language. In an example, the output data may comprise a data structure describing anatomical model if the LLM algorithm is asked (e.g., as it input) to display an anatomical model of a particular anatomical structure. In another example, the output data may comprise a modified data structure describing the anatomical model if the LLM algorithm is asked to modify or manipulate an existing anatomical model, for example being displayed to a user. In another example, the output data may comprise one or more elements of natural language if the LLM algorithm is asked to describe the anatomical model in some way.

Fig. 9 is a flowchart of a further example of a computer-implemented method 900 of processing a data structure using an LLM algorithm. The method 900 comprises, at step 902, obtaining input data, the input data comprising a data structure as disclosed herein. At step 904, the method 900 comprises processing the input data with an LLM algorithm to generate output data comprising at least one of (i) a modified data structure describing the anatomical model; and (ii) one or more elements of natural language. In this example, a data structure may be provided as an input to the LLM algorithm, and the LLM algorithm may generate as it output a description of the anatomical model, for example, or a modified version of the data structure.

According to a further aspect, the present invention provides a method of training an LLM algorithm. Fig. 10 is a flowchart of an example of a computer-implemented method 1000 of training and LLM algorithm. The method 1000 comprises, at step 1002, obtaining input training data comprising a plurality of data structures as disclosed herein. At step 1004, the method 1000 comprises creating training data from the input training data. At step 1006, the method 1000 comprises training the LLM algorithm using the training data to obtain a trained LLM algorithm. In some embodiments, a self-supervised training mechanism may be used for training the LLM algorithm. In other embodiments, the LLM algorithm may be trained, for example, by providing one or more elements of natural language along with an example of an intended output. For example, training data may include a command, such as "shift plane more posteriorly" and an example of an anatomical model in which the plane has been moved posteriorly.

In some embodiments, the training data may comprise a plurality of training inputs and a respective plurality of corresponding training outputs, wherein each training input comprises (i) one or more first elements of natural language and (ii) a data structure describing an anatomical model, and wherein the corresponding training output comprises (i) one or more second elements of natural language and/or (ii) a modified data structure describing the anatomical model.

In general, therefore, the present disclosure provides a mechanism for encoding an anatomical model in such a way that it can be used can be processed using an LLM algorithm (e.g., provided as an input to the LLM algorithm and/or modified and provided as an output). For example, an output may comprise a version of the anatomical model in a particular orientation or from a particular perspective. In another example, the LLM algorithm may be trained for a downstream task that correlates natural language descriptions of anatomical regions with the respective triangle regions in one or several "digital twin" models. In other words, a "digital twin" (e.g., replica) model of a subject could be created and used to investigate current of historic medical records of the subject. This may, for example, be used to parse through medical records and mark regions in the anatomical model corresponding to certain findings. In another example, the LLM algorithm may provide as its output wording describing the anatomical model.

The processor 502 can comprise one or more processors, processing units, multi-core processors or modules that are configured or programmed to control the apparatus 500 in the manner described herein. In particular implementations, the processor 502 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g., Java interpreter instructions). Alternatively, one or more or all of the sub-routines may be stored in at least one external library file and linked with a main program either statically or dynamically, e.g., at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (200) for encoding an anatomical model of an anatomical structure of a subject into a data structure suitable for processing using a large language model, LLM, algorithm, the method comprising:
obtaining (202) an anatomical model of the anatomical structure of the subject, the anatomical model comprising a plurality of model elements;
converting (204) a first set of model elements of the plurality of model elements of the anatomical model into a first vector representation capable of being processed using an LLM algorithm; and
composing (206) a data structure representative of the plurality of model elements of the anatomical model based on the first vector representation;
wherein the data structure comprises an encoded version of the anatomical model of the anatomical structure configured to be processed using the LLM algorithm.

2. A computer-implemented method (200, 400) according to claim 1, further comprising:
converting (402) a second set of model elements of the plurality of model elements of the anatomical model into a second vector representation capable of being processed using an LLM algorithm;
wherein composing the data structure representative of the plurality of model elements of the anatomical model comprises concatenating the first vector representation and the second vector representation.

3. A computer-implemented method (200, 400) according to claim 1 or claim 2, further comprising:
obtaining (404), from at least one of: i) a user input; and ii) the anatomical model, at least one data string to be included in the data structure, the at least one data string being associated with the anatomical model.

4. A computer-implemented method (200, 400) according to claim 3, further comprising:
appending (406) the at least one data string as a header of the first vector representation.

5. A computer-implemented method (200, 400) according to claim 3 or claim 4, wherein the at least one data string comprises an indication of the nature of the first vector representation in the data structure.

6. A computer-implemented method (200, 400) according any of claims 3 to 5, wherein the at least one data string comprises data indicative of a type of anatomical structure represented by the anatomical model.

7. A computer-implemented method (200, 400) according to any of claims 3 to 6, further comprising:
converting (408) the at least one data string into an additional vector representation capable of being processed using an LLM algorithm;
wherein the at least one data string comprises one or more offset values to distinguish the first vector representation from the additional vector representation in the data structure.

8. An apparatus (500) for encoding an anatomical model of an anatomical structure of a subject into a data structure suitable for processing using a large language model, LLM, algorithm, the apparatus comprising:
a processor (402) configured to perform a method according to any of the preceding claims.

9. A data structure (600) comprising information describing an anatomical model, wherein the information is encoded into a first vector representation for processing using a large language model, LLM, algorithm;
wherein the first vector representation comprises a plurality of vectors, each vector representing a corresponding one of a plurality of model elements of the anatomical model.

10. A computer-implemented method (700) of processing a data structure using a large language model, LLM, algorithm, the method comprising:
obtaining (702) input data, the input data comprising (i) one or more elements of natural language and (ii) a data structure according to claim 9;
processing (704) the input data using an LLM algorithm to generate output data.

11. A computer-implemented method (800) of processing a data structure using a large language model, LLM, algorithm, the method comprising:
obtaining (802) input data comprising one or more elements of natural language;
processing (804) the input data with an LLM algorithm to generate output data, wherein the LLM algorithm has been trained using training data comprising a plurality of data structures according to claim 9, each data structure describing a respective anatomical model.

12. A computer-implemented method (700, 800) according to claim 10 or claim 11, wherein the output data comprises at least one of (i) a data structure describing an anatomical model; ii) a modified data structure describing the anatomical model and (iii) one or more elements of natural language.

13. A computer-implemented method (900) of processing a data structure using a large language model, LLM, algorithm, the method comprising:
obtaining (902) input data, the input data comprising a data structure according to claim 9; and
processing (904) the input data with an LLM algorithm to generate output data comprising at least one of (i) a modified data structure describing the anatomical model; and (ii) one or more elements of natural language.

14. A computer-implemented method (1000) of training a large language model, LLM, algorithm, the method comprising:
obtaining (1002) input training data comprising a plurality of data structures according to claim 9;
creating (1004) training data from the input training data; and
training (1006) the LLM algorithm using the training data to obtain a trained LLM algorithm.

15. A computer-implemented method according to claim 14, wherein the training data further comprises a plurality of training inputs and a respective plurality of corresponding training outputs, wherein each training input comprises (i) one or more first elements of natural language and (ii) a data structure describing an anatomical model, and wherein the corresponding training output comprises (i) one or more second elements of natural language and/or (ii) a modified data structure describing the anatomical model.
